# EUROPÄISCHE PATENTSCHRIFT

(11) **EP 0 427 827 B1**
(45) Veröffentlichungstag und Bekanntmachung des Hinweises auf die Patenterteilung: **15.01.1997**
(21) Anmeldenummer: 90906872.8
(22) Anmeldetag: 10.05.1990
(51) Int. Cl.: C07D 457/12, A61K 31/48

(54) **8-alpha-ACYLAMINO-ERGOLINE, IHRE HERSTELLUNG UND VERWENDUNG ALS ARZNEIMITTEL**
8-alpha-ACYLAMINO-ERGOLINES, THEIR PREPARATION AND USE AS DRUGS
8-alpha-ACYLAMINO-ERGOLINES, LEUR PRODUCTION ET LEUR UTILISATION EN TANT QUE MEDICAMENTS

(30) Priorität: 12.05.1989 DE 3915950
(43) Veröffentlichungstag der Anmeldung: 22.05.1991
(73) Patentinhaber: SCHERING AKTIENGESELLSCHAFT, 13342 Berlin (DE)
(72) Erfinder: SAUER, Gerhard, D-1000 Berlin 28 (DE); BRUMBY, Thomas, D-1000 Berlin 62 (DE); WACHTEL, Helmut, D-1000 Berlin 19 (DE); TURNER, Jonathan, D-1000 Berlin 28 (DE); LÖSCHMANN, Peter, Andreas, D-1000 Berlin 19 (DE)
(86) Internationale Anmeldenummer: DE9000339
(87) Internationale Veröffentlichungsnummer: WO9013550

(56) Entgegenhaltungen:
- EP-A- 0 160 842
- EP-A- 0 250 357
- CH-A- 615 929
- DE-A- 3 500 251

## Beschreibung

Die Erfindung betrifft 8α-Acylaminoergoline, ihre Herstellung und Verwendung als Arzneimittel und Zwischenverbindungen zur Herstellung von 8α-Acylaminoergolinen.

In der DOS 35 00 251 werden 8α-Acylamino-Ergoline beschrieben, die prolactinsekretionshemmende Wirkung zeigen und, falls in 6-Stellung längerkettige Alkylreste vorhanden sind, insbesondere die Sekretion des luteinisierenden Hormons hemmen. 8α-Diethylharnstoff- und thioharnstoff-Ergolinderivate, die in 6-Stellung einen längerkettigen Kohlenwasserstoffrest besitzen, werden in der EP-A-351 352 beschrieben.

Ergolinderivate, die in 2-Stellung substituiert sind, sind aus EP-A-0160 842 bekannt.

Die erfindungsgemäß in 6-Stellung mit einem längerkettigen Kohlenwasserstoffrest substituierten 8α-Acylaminoergolinderivate zeigen im Vergleich mit den 6-Methyl-Derivaten eine verminderte Apomorphin-antagonistische Aktivität und gesteigerte Dopamin-agonistische Aktivität. Gleichzeitig bleibt die metabolische Stabilität der Verbindung erhalten bzw. wird verbessert.

Die Erfindung betrifft Verbindungen der Formel I 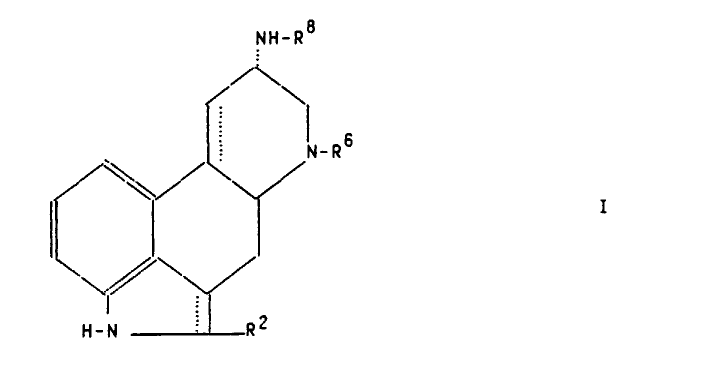 worin
C 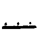 C jeweils eine Einfach- oder Doppelbindung,
R² gegebenenfalls substituiertes C₁₋₇-Alkyl, C₂₋₇-Alkenyl, CH₂-O-C₁₋₄Alkyl oder CH₂-S-C₁₋₄-Alkyl.
R⁶ C₂₋₆-Alkyl, C₃₋₆-Alkenyl oder C₃₋₅-Cycloalkyl-C₁₋₂-alkyl und
R⁸ -CXR³ oder -SO₂R⁵ ist.
worin R³ Wasserstoff, gegebenenfalls substituiertes C₁₋₆-Alkyl. S-C₁₋₆-Alkyl, -O-(CH₂)ₙ-N(CH₃)₂ oder NR⁹R¹⁰ ist, X Sauerstoff oder Schwefel ist und R⁵ CH₃ oder eine gegebenenfalls mit C₁₋₄-Alkyl mono- oder disubstituierte Aminogruppe bedeutet, worin R⁹ und R¹⁰ jeweils Wasserstoff, C₁₋₄-Alkyl, C₃₋₄-Alkenyl, -(CH₂)ₙN(CH₃)₂, CH₂CF₃ oder gemeinsam mit dem Stickstoffatom einen 5-6 gliedrigen gesättigten oder ungesättigten Heterocyclus bilden, der durch ein bis zwei weitere Heteroatome unterbrochen sein kann und n = 1,2,3 oder 4 ist sowie deren Säureadditionssalze, wobei R⁸ nicht CXN (C₂H₅)₂ bedeutet und falls R⁸-CO-C(CH₃)₃ und R⁶ n-Propyl und R² Methyl ist, C₂ 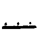 C₃ eine Einfachbindung bedeutet.

Unter Alkyl ist jeweils ein geradkettiger oder verzweigter Alkylrest zu verstehen wie beispielsweise Methyl, Ethyl, n-Propyl, Isopropyl, n-Butyl, Isobutyl, sek. Butyl, tert. Butyl, Pentyl, Hexyl, Heptyl, 2,2-Dimethylpropyl, 2-Methylbutyl, 3-Methylbutyl, 1-Ethylbutyl, Isopentyl, Isoheptyl, 1-Methyl-1ethyl-propyl.

Der Alkylrest R² kann insbesondere in 1-Stellung substituiert sein mit einer Hydroxy-, C₁₋₄ -Alkoxy- oder C₂₋₅-Acyloxy-Gruppe entsprechend der Formel -C(OR')R"R''', worin R" und R''' jeweils Wasserstoff oder Alkylreste mit max. 6 Kohlenwasserstoffatomen bedeuten und R' insbesondere Wasserstoff oder Acetyl ist oder eine S-C₁₋₄-Alkyl-Gruppe.

Als Acylgruppen sind aliphatische Carbonsäuren geeignet wie beispielsweise Essigsäure, Propionsäure, Buttersäure, Capronsäure und Trimethylessigsäure.

Bedeuten R² oder R⁶ einen Alkenylrest, so enthält dieser bevorzugt nur eine Doppelbindung, wobei die Doppelbindung im Rest R⁶ nicht benachbart vom Stickstoffatom stehen kann. Als Alkenylreste sind beispielsweise geeignet: Vinyl, 1-Propenyl, 2-Propenyl, 1-Methyl-2-propenyl, 1-Butenyl, Methallyl, Allyl.

Bedeutet R⁶ eine Cycloalkyl-alkyl-Gruppe, so ist beispielsweise Cyclopropylmethyl,Cyclopropylethyl, Cyclobutylethyl, Cyclopentylmethyl, Cyclopentylethyl gemeint.

Der Alkylrest R³ kann ein- oder zweifach substituiert sein mit Hydroxy-, C₁₋₄-Alkoxy-, Acetyloxy- oder Dimethylaminogruppen, wobei Alkylgruppen mit bis zu 3 Kohlenstoffatomen und einem Substituenten bevorzugt sind. Bilden R⁹ und R¹⁰ gemeinsam mit dem Stickstoffatom einen gesättigten oder ungesättigten Heterocyclus, der durch ein bis zwei weitere Heteroatome unterbrochen sein kann, so kann dieser durch Sauerstoff, Schwefel oder eine gegebenenfalls mit C₁₋₄ Alkyl oder Phenyl substituierte NH-Gruppe oder durch 2-Stickstoffatome unterbrochen sein. Beispielsweise kommen Morpholin, Thiomorpholin, Piperidin, Imidazolidin, Piperazin, Pyrrolidin, Pyrazolidin, Imidazol, Triazol u.a. in Betracht.

Als bevorzugte Ausführungsformen fur R⁶ ist C₂₋₄-Alkyl, C₃₋₄-Alkenyl oder Cycloalkylalkyl mit bis zu 5 Kohlenstoffatomen zu betrachten und fur R² C₁₋₄-Alkyle und C₂₋₄-Alkenyle.

Die Verbindungen der Formel I können als E- oder Z-Isomere oder, falls ein chirales Zentrum im Rest R² vorhanden ist, als Diastereomere und als Gemische derselben auftreten. Die Isomeren und Isomerengemische sind von der vorliegenden Erfindung auch umfaßt. Die physiologisch verträglichen Saureadditionssalze leiten sich von den bekannten anorganischen und organischen Säuren ab wie zum Beispiel Salzsäure, Schwefelsäure, Bromwasserstoffsäure, Zitronensaure, Maleinsaure, Fumarsäure, Weinsäure u.a..

Die Verbindungen der Formel I sowie ihre Säureadditionssalze weisen insbesondere zentral dopaminerge Wirksamkeit auf und sind daher als Arzneimittel verwendbar.

Die dopaminagonistische Wirkung wurde mit Hilfe der Methode nach Horowski, R. und Wachtel, H. Eur. J. Pharmaz. col. 36: 373-383, 1976 bestimmt: Eine Stunde nach i.p.-Vorbehandlung mit Prüfsubstanz bzw. Vehikel wurde an Ratten das Vorhandensein von Verhaltensstereotypien (Kau-, Leck- oder Nagebewegungen) während 2 min (60.-62. min p.i.) ermittelt. Tiere, die während des 2 Minutenintervalls Kau-, Leck- oder Nagebewegungen zeigten, wurden als stereotyp betrachtet.

Die Ergebnisse sind in der Tabelle 1 dargelegt:

**Tabelle 1**

| | | | |
|---|---|---|---|
| Sterotypien-auslösende Wirksamkeit an Ratten 1 Stunde nach i.p.-Vorbehandlung. Effektive Dosen (ED₅₀) mit 95 %-Vertrauensbereich wurden mit Hilfe der Probit-Analyse ermittelt. n = Anzahl der Tiere | | | |

| | | Wirksamkeit | |
|---|---|---|---|
| Verbindung | n | ED₅₀ | 95%-Vertrauensbereich |
| A | 8 | 0,080 | 0,010 - 0,170 |
| B | 8 | 0,050 | 0,034 - 0,079 |
| A = N-(2-Methyl-6-n-propyl-8α-ergolinyl)-dithiocarbamidsäuremethylester | | | |
| B = N-(2-Methyl-6-n-propyl-8α-ergolinyl)-thioformamid | | | |

Da sie sich insbesondere durch dopaminagonistische Wirkung auszeichnen, ohne daß α-adrenerge Effekte sich bemerkbar machen, eignen sich die erfindungsgemäßen Verbindungen zur Behandlung von Dopaminmangelzuständen in Lebewesen und insbesondere von Morbus Parkinson.

Zur Verwendung der erfindungsgemäßen Verbindungen als Arzneimittel werden diese in die Form eines pharmazeutischen Präparats gebracht, das neben dem Wirkstoff für die enterale oder parenterale Applikation geeignete pharmazeutische, organische oder anorganische inerte Trägermaterialien, wie zum Beispiel Wasser, Gelatine, Gummi arabicum, Milchzucker, Stärke, Magnesiumstearat, Talk, pflanzliche Ole, Polyalkylenglykole usw. enthält. Die pharmazeutischen Präparate können in fester Form, zum Beispiel als Tabletten, Dragees, Suppositorien, Kapseln oder in flüssiger Form, zum Beispiel als Lösungen, Suspensionen oder Emulsionen vorliegen. Gegebenenfalls enthalten sie darüber hinaus Hilfsstoffe wie Konservierungs-, Stabilisierungs-, Netzmittel oder Emulgatoren, Salze zur Veränderung des osmotischen Drucks oder Puffer.

Die erfindungsgemäßen Verbindungen werden in einer Dosis von 0.001 bis 10 mg aktiver Substanz in einen physiologisch verträglichen Träger eingebracht. Die Anwendung der erfindungsgemäßen Verbindungen erfolgt in einer Dosis von 0,00001 bis 0.1 mg/kg/Tag, vorzugsweise 0,001 bis 0,1 mg/kg/Tag analog dem bekannten Mittel Bromcryptin.

Die Herstellung der erfindungsgemäßen Verbindungen der Formel I kann nach an sich bekannten Methoden durchgeführt werden.

Beispielsweise gelangt man zu Verbindungen der Formel I gemäß Anspruch 1, indem man
a) eine Verbindung der Formel II 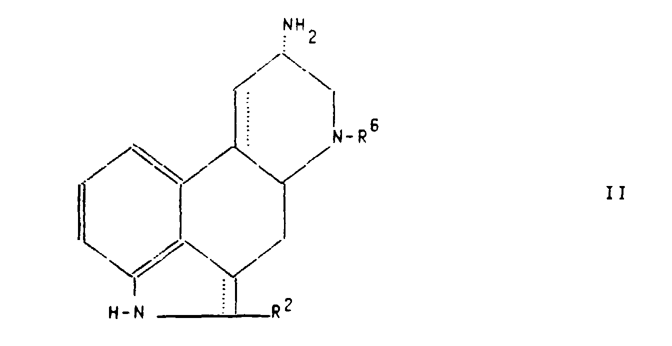 worin R² und R⁶ die obige Bedeutung haben, mit einer Säure oder deren funktionellem Derivat acyliert,
b) eine Verbindung der Formel II und eine Verbindung der Formel III

   R⁹-N=C=X III

   worin R⁹ und X die obige Bedeutung haben, R⁹ jedoch nicht Wasserstoff sein kann, addiert zu einer Verbindung mit R⁸ in der Bedeutung von -CX-NHR⁹
c) eine Verbindung der Formel IV 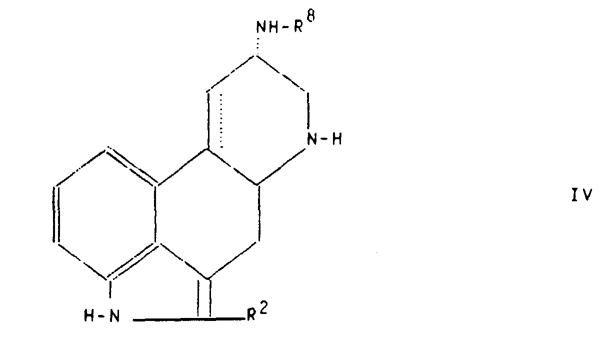 worin R² und R⁸ die obige Bedeutung haben, alkyliert oder alkenyliert
d) eine Verbindung der Formel V 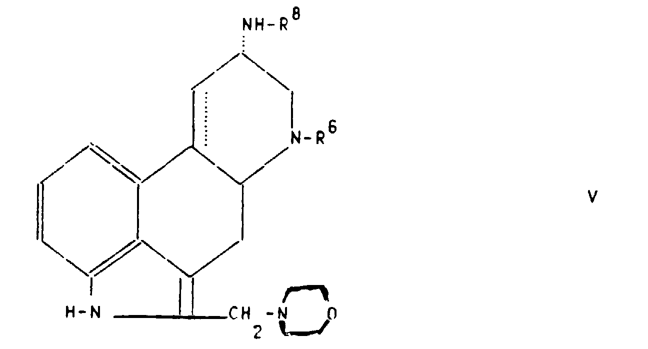 worin R⁶ und R⁸ die obige Bedeutung haben in 2-Stellung substituiert
e) eine Verbindung der Formel VI 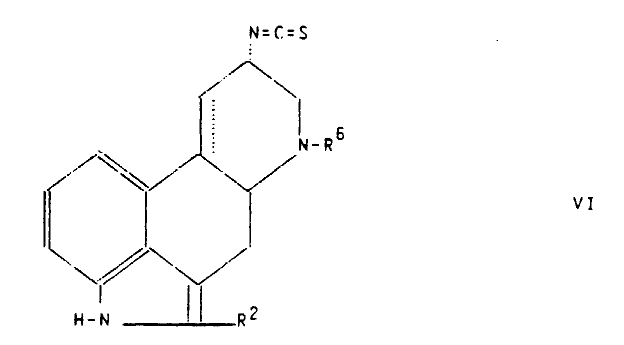 worin R² und R⁶ die oben genannte Bedeutung haben mit einem Nukleophil umsetzt zu Verbindungen der Formel I mit R³ = -S-C₁₋₆-Alkyl, -O-(CH₂)ₙ- N(CH₃)₂ oder NR⁹R¹⁰, worin R⁹, R¹⁰ und n die oben genannte Bedeutung haben und gewunschtenfalls anschließend eine C 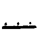 CEinfachbindung oxidiert oder eine C 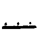 C-Doppelbindung reduziert oder eine Carbonylgruppe thioliert oder die Säureadditionssalze bildet.

Die Acylierung der Verbindungen der Formel I nach dem Verfahren a) kann in üblicher Weise vorgenommen werden. So kann man die freie Säure R⁸OH oder deren reaktive Derivate wie Halogenide oder Anhydride gegebenenfalls in Gegenwart einer Base in einem geeigneten inerten Lösungsmittel bei Temperaturen von Raumtemperatur bis zur Siedetemperatur des Lösungsgemisches mit dem Amin der Formel II umsetzen. Als Lösungsmittel sind Ether wie Tetrahydrofuran, Dioxan, chlorierte Kohlenwasserstoffe wie Dichlormethan oder aprotische Lösungsmittel wie DMF geeignet. Bei Verwendung eines Anhydrids zur Acylierung kann auch überschüssiges Anhydrid als Lösungsmittel eingesetzt werden. Die Formylierung wird zweckmäßigerweise mit einem gemischten Anhydrid von Essigsäure und Ameisensäure durchgeführt. Als Basen sind beispielsweise Triethylamin, Hünig-Basen oder 2,6-Lutidin geeignet oder man führt die Acylierung in Pyridin als Lösungsmittel durch. Die Herstellung der Harnstoff- und Thioharnstoffderivate kann beispielsweise über reaktive Imidazolide, die aus N,N-Carbonyldiimidazol bzw. N,N-Thiocarbonyldiimidazol und primären und sekundären Aminen R⁹R¹⁰NH erhalten werden, erfolgen. Auch durch Umsetzung mit Carbamoylchloriden R⁹R¹⁰NCO-Cl können Harnstoffderivate dargestellt werden.

Die Addition von Aminen an Isocyanate oder Isothiocyanate nach Verfahren b) kann nach der in EP-82 808 beschriebenen Methode erfolgen, indem man beispielsweise in einem inerten Lösungsmittel wie Kohlenwasserstoffen, chlorierten Kohlenwasserstoffen, Ethern oder Estern beispielsweise in Hexan, Toluol, Dichlormethan, Diethylether, Essigsaureethylester u.a. bei Raumtemperatur oder erhöhter Temperatur die Umsetzung vornimmt.

Die Einführung der Substituenten in 6- oder in 2-Stellung kann vor oder nach der Acylierung in 8-Stellung erfolgen.

Die Substitution in 6-Stellung nach dem Verfahren c) kann beispielsweise nach A. Cerny et al. Coll. Czech. Chem. Comm. 49, 2828 (1984) oder nach dem in der EP-21 206 beschriebenen Verfahren durchgeführt werden, indem man die 6H-Verbindung der Formel IV mit den entsprechenden R⁶-Halogeniden (Bromiden, Chloriden, Iodiden) umsetzt. Zweckmäßigerweise erfolgt die Reaktion in einem inerten Lösungsmittel wie Dimethylsulfoxid, Dimethylformamid, Acetonitril oder Nitromethan in Gegenwart von Basen wie Alkalihydroxiden oder -carbonaten.

Die Einführung des Substituenten R² kann beispielsweise nach den in der EP-A-351 352 beschriebenen Verfahren erfolgen. Hierbei kann die Mannichbase der Formel V oder deren Quartärsalz nucleophil substituiert werden oder über das 2-Aldehyd-Derivat als Zwischenverbindung der gewünschte Substituent R² eingeführt werden. Der nucleophile Austausch erfolgt gegebenenfalls nach Quartärnisierung der Mannichbase in einem polaren, protischen oder aprotischen Lösungsmittel wie Alkoholen, Ethern oder chlorierten Kohlenwasserstoffen bei Raumtemperatur oder erhöhter Temperatur, wobei als nucleophile Anionen Alkoholate oder Thiolate eingesetzt werden können, die gewünschtenfalls anschließend in die CH₂-OHGruppe überführt werden können. Zur Herstellung des 2-Methyl-Derivates kann das Quartarsalz der Formel V in polaren Lösungsmitteln wie Alkoholen mit Natriumborhydrid reduziert werden.

Die Oxidation zu einer 2-CHO-Verbindung kann analog dem in R.A. Jones et al. Synthetic Communications 16, 1799 (1986) beschriebenen Verfahren mit Braunstein oder tert. Butylhypochlorit in inerten Lösungsmitteln bei Raumtemperatur erfolgen. Die Umwandlung der 2-Formyl-Verbindungen zu Verbindungen der Formel I, worin R² einen Alkenylrest bedeutet, kann in einer Wittigreaktion erfolgen, wie beispielsweise mit Alkyl-triphenylphosphonium-halogenid in polaren Lösungsmitteln wie cyclischen und acyclischen Ethern, chlorierten Kohlenwasserstoffen, Dimethylformamid oder Dimethylsulfoxid bei Temperaturen von -50 °C bis zur Siedetemperatur des Reaktionsgemisches vorgenommen werden, wobei zur Erzeugung des Ylens starke Basen wie Alkalialkoholate, Lithiumorganyl u.a. zugesetzt werden.

Die Darstellung von in 1-Stellung hydroxylierten Substituenten R² kann beispielsweise durch Reaktion der Aldehyde und Ketone mit Grignard-Verbindungen oder Lithium-Organylen erfolgen. Die Grignardierung kann mit den üblichen Grignard-Reagenzen wie Alkylmagnesiumhalogeniden in einem aprotischen Lösungsmittel wie cyclischen und acyclischen Ethern bei tiefen Temperaturen (-70 °C bis 0 °C) erfolgen. Die Umsetzung mit Alkyl-Lithium erfolgt unter analogen Bedingungen.

Die Acetylierung einer Hydroxylgruppe kann nach den üblichen Methoden erfolgen wie beispielsweise durch Umsetzung mit Säureanhydriden oder Säurechloriden.

Enthält der Substituent R² eine Hydroxylgruppe, so kann diese beispielsweise durch Umsetzung mit Na BH₄ in Eisessig zum entsprechenden 2-Alkylderivat reduziert werden oder mit Braunstein oxidiert zum Keton oder dehydratisiert werden unter Einführung einer Doppelbindung. Enthält der Substituent R² eine Doppelbindung, so kann diese beispielsweise katalytisch reduziert werden. Die Einführung des Substituenten R² -C(OH)R"R"' kann wie oben beschrieben durch Reaktion des Ketons mit Grignard-Verbindungen oder Lithium-Organylen erfolgen.

Werden die Verbindungen der Formel I nach Verfahren e) dargestellt, so sind für die Umsetzung die unter Verfahrensvariante b) genannten Rektionsbedingungen geeignet. Als Nukleophile können Alkohole, primäre und sekundäre Amine und Merkaptane der Formel H-R³ eingesetzt werden, wobei R³ -S-C₁₋₆-Alkyl, -0-(CH₂)ₙ-N(CH₃)₂ oder NR⁹R¹⁰ bedeutet.

Die fakultativ nachfolgende stereoselektive Hydrierung der 2,3-Doppelbindung kann beispielsweise nach den in der EP-A-286 575 beschriebenen Verfahren in Gegenwart einer Säure mit Organylsilanen oder mit Wasserstoff/Edelmetallkatalysatoren erfolgen.

Soll fakultativ die Einführung der C₂-C₃-Doppelbindung vorgenommen werden, so kann dies beispielsweise nach den im EP-A-190534 beschriebenen Verfahren mit tert. Butylhypochlorit oder mit MnO₂ nach EP-A-118849 vorgegangen werden.

Die Uberführung der Amide und Harnstoffderivate in die Thioamide und Thioharnstoffderivate kann beispielsweise nach dem in der EP-A-217 730 beschriebenen Verfahren durch Umsetzung mit Phosphoroxychlorid und einem Thiolierungsmittel oder mit Lawesson-Reagenz nach Fieser and Fieser Reagents for Org. Synth. IX, 49 erfolgen. Die Verbindungen der Formel I werden entweder als freie Basen oder in Form ihrer physiologisch verträglichen Säureadditionssalze isoliert.

Zur Bildung von Salzen wird eine Verbindung der Formel I beispielsweise in wenig Methanol oder Methylenchlorid gelöst und mit einer konzentrierten Lösung der gewunschten Säure versetzt.

Die Isomerengemische können nach den üblichen Methoden wie beispielsweise Kristallisation, Chromatographie oder Salzbildung in die Diastereomeren bzw. E/Z-Isomeren aufgetrennt werden.

Die Erfindung umfaßt auch Verbindungen der Formel II 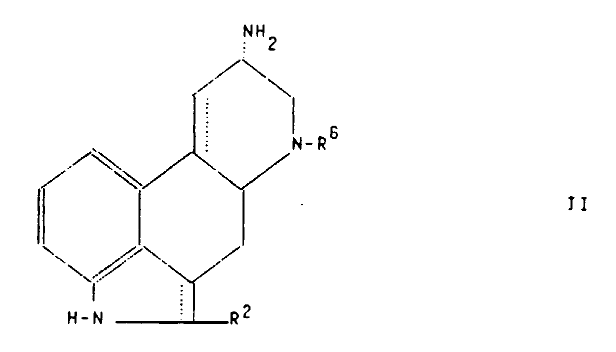 worin R² und R⁶ die obige Bedeutung haben und Verbindungen der Formel VI 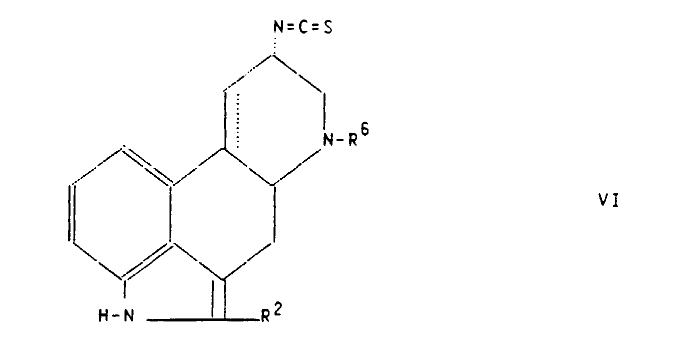 worin R² und R⁶ die obige Bedeutung haben.

Diese Verbindungen sind wertvolle Zwischenprodukte zur Herstellung pharmakologisch wirksamer Verbindungen. Die Umwandlung der Zwischenprodukte in die Wirksubstanzen erfolgt nach den oben beschriebenen Verfahren.

Soweit die Herstellung der Ausgangsverbindungen nicht beschrieben wird, sind diese bekannt oder analog zu bekannten Verbindungen oder hier beschriebenen Verfahren herstellbar.

Die nachfolgenden Beispiele sollen das erfindungsgemäße Verfahren erläutern.

### Ausgangsverbindungen

### Darstellung von 2,6-disubstituierten 8α-Amino-ergolinen

1.) Man bereitet sich eine Lösung von 25 mmol Lithiumdiisopropylamid in 50 ml Tetrahydrofuran und gibt die Lösung von 3,38 g 6-n-Propyl-2-vinyl-8β-ergolincarbonsäuremethylester (10 mmol) in 50 ml Tetrahydrofuran bei - 30 °C zu, rührt 30 Minuten bei dieser Temperatur und säuert dann mit 10%iger Salzsäure an. Nach dem Erwärmen auf Raumtemperatur wird mit gesättigter Bicarbonatlösung versetzt, mit Dichlormethan ausgeschüttelt und die organische Phase abgetrennt, getrocknet und eingedampft. Der Rückstand wird an Kieselgel mit Dichlormethan/Methanol chromatographiert, Ausbeute an 6-n-Propyl-2-vinyl-8α-ergolincarbonsäuremethylester 2,0 g (60 % der Theorie).

Ohne Kristallisation wird der 8α-Ester mit Hydrazinhydrat/Hydrazinhydrochlorid in Propanol in das entsprechende Hydrazin überführt, mit Natriumnitrit in salzsaurer Lösung zum 8α-Ergolincarbonsäureazid nitrosiert und das Azid bei 80 °C umgelagert. Nach Abkühlen wird wie oben beschrieben aufgearbeitet und der Rückstand kristallisiert, Ausbeute 1, 33 g (45 % der Theorie) 6-n-Propyl-2-vinyl-8α-ergolinylamin.

Analog können weitere substituierte Ergolinylamine dargestellt werden.

2.) 4,35 g 1,1-Diethyl-3-(2-methyl-6-n-propyl-8α-ergolinyl)-harnstoff (11 mmol) werden in 1N HCl suspendiert und 16 Stunden auf 120 °C erhitzt. Die Lösung läßt man abkühlen, macht vorsichtig mit Ammoniaklösung alkalisch und extrahiert mit Dichlormethan. Der Rückstand wird aus Dichlormethan/Methanol/Diisopropylether kristallisiert, Ausbeute 2,69 g (83 % der Theorie) 2-Methyl-6-n-propyl-8α-ergolinylamin, [α]_{D} = -82 °
(0,5 % in Chloroform)

Analog werden aus den entsprechenden Ergolinylharnstoffen oder Ergolinylthioharnstoffen die folgenden Ergolinylamine dargestellt:
6-Ethyl-2-methyl-8α-ergolinylamin
   Ausbeute 74 %
6-Allyl-2-methyl-8α-ergolinylamin
   Ausbeute 53 %
6-Cyclopropylmethyl-2-methyl-8α-ergolinylamin
   Ausbeute 59 %
2,6-Diethyl-8α-ergolinylamin
   Ausbeute 76 %
2-Ethyl-6-n-propyl-8α-ergolinylamin
   Ausbeute 81 %
2-Ethyl-6-allyl-8α-ergolinylamin
   Ausbeute 71 %
6-Ethyl-2-morpholinomethyl-8α-ergolinylamin
   Ausbeute 71 %
2-Morpholinomethyl-6-n-propyl-8α-ergolinylamin
   Ausbeute 80 %

3.) Man löst 880 mg 3-(9,10-Didehydro-2-methyl-6-n-propyl-8α-ergolinyl)-1,1diethylharstoff (2,3 mmol) in 30 ml Trifluoressigsäure und gibt bei Raumtemperatur 1,48 ml Triethylsilan in drei gleichen Portionen im Abstand von 5 Minuten zu. Dann rührt man 60 Minuten, fügt erst Eis und anschließend 25%ige Ammoniaklösung unter Kühlen zu und schüttelt die alkalische Lösung mit Dichlormethan aus. Die organischen Phasen werden getrocknet und eingedampft, Ausbeute nach Chromatographie 378 mg. Durch Kristallisation aus Essigester/Diisopropylether können 106 mg des 2,3Dihydro-Derivates erhalten werden, [α]_{D} = + 204 ° (0,5 % in Chloroform)

271 mg des Rohproduktes werden in 40 ml 4N HCl 16 Stunden auf 110 °C erhitzt. Nach dem Abkühlen versetzt man mit Eis, macht mit Ammoniak alkalisch und schüttelt mit Dichlormethan aus. Das Rohprodukt wird aus Essigester/Diisopropylether kristallisiert, Ausbeute 46 mg (23 % der Theorie) 9,10-Didehydro-2,3β-diyhdro-2β-methyl-6-n-propyl-8α-ergolinylamin, [α]_{D} = + 81 ° (0,2 % in Chloroform).

Analog werden aus den entsprechenden 9,10-Didehydro-ergolinylharnstoffen die folgenden 9,10-Didehydro-2,3β-dihydro-8α-ergolinylamine dargestellt:
9,10-Didehydro-2,3β-dihydro-6-ethyl-2β-methyl-8α-ergolinylamin
   Ausbeute 31 %
6-Allyl-9,10-didehydro-2,3β-diyhdro-2β-methyl-8α-ergolinylamin
   Ausbeute 19 %
6-Cyclopropylmethyl-9,10-didehydro-2,3-dihydro-2β-methyl-8α-ergolinylamin
   Ausbeute 32 %
9,10-Didehydro-2,3β-dihydro-2,6-diethyl-8α-ergolinylamin
   Ausbeute 41 %
9,10-Didehydro-2,3-didehydro-2-ethyl-6-n-propyl-8α-ergolinylamin
   Ausbeute 35 %
6-Allyl-9,10-didehydro-2,3β-dihydro-2-ethyl-8α-ergolinylamin
   Ausbeute 49 %

Nach der gleichen Vorschrift lassen sich 2,3β-Dihydro-8α-ergolinylamine aus den entsprechenden Ergolinylharnstoffen darstellen.
2,3β-Dihydro-2methyl-6-n-proypl-8α-ergolinylamin
   Ausbeute 65 %.
2,3β-Didehydro-6-ethyl-2-methyl-8α-ergolinylamin
   Ausbeute 73 %
6-Allyl-2,3β-diyhdro-2-methyl-8α-ergolinylamin
   Ausbeute 63 %
2,3-Dihydro-2-ethyl-6-n-propyl-8α-ergolinylamin
   Ausbeute 78 %

### Beispiel 1

### (Dimethylamino)-(2-methyl-6-n-propyl-8α-ergolinylamino)-sulfon

Bei Raumtemperatur mischt man 30 ml Dichlormethan, 10 ml 2,6-Lutidin und 1 ml Dimethylaminosulfonsäurechlorid (10 mmol). Dazu gibt man die Suspension von 300 mg 2-Methyl-6-n-propyl-8α-ergolinylamin (1,06 mmol) in 30 ml Dichlormethan und rührt 16 Stunden bei Raumtemperatur und anschließend 2 Stunden bei 50 °C. Die Reaktionslösung wird dreimal mit je 200 ml gesättigter Kupfersulfatlösung geschüttelt, die organische Phase mit Wasser gewaschen, getrocknet und eingedampft, zuletzt am Hockvakuum. Das Rohprodukt wird an Kieselgel mit Dichlormethan/Methanol chromatographiert und aus Essigester/Diisopropylether/Hexan kristallisiert, Ausbeute 73 mg (17 % der Theorie), [α]_{D} = - 44,9 ° (0,5 % in Chloroform).

Analog wird dargestellt mit Diethylaminosulfonsäurechlorid:
(Diethylamino)-(2-methyl-6-n-propyl-8α-ergolinylamino)-sulfon Ausbeute 25 % als weinsaures Salz, [α]_{D} = - 34 ° (0,5% in Methanol).

### Beispiel 2

Analog Beispiel 1 wird aus 9,10-Didehydro-2,3β-dihydro-2-methyl-6-n-propyl-8αergolinylamin dargestellt:
(9,10-Didehydro-2,3β-dihyro-2-methyl-6-n-propyl-8α-ergolinylamino)-dimethylaminosulfon, Ausbeute 37 %.

### Beispiel 3

### (9,10-Didehydro-2-methyl-6-n-propyl-8α-ergolinyl)-dimethylamino-sulfon

390 mg (9,10-Didehydro-2,3β-dihyro-2-methyl-6-n-propyl-8α-ergolinylamino)dimethylamino-sulfon (1 mol) wird in 30 ml wasserfreiem Tetrahydrofuran und 0,5 ml Triethylamin gelöst und die Mischung auf - 40 °C abgekühlt. Man gibt 0,16 ml tert.-Butylhypochlorit (1,34 mmol) in 10 wasserfreiem Tetrahydrofuran tropfenweise zu und rührt 30 Minuten bei - 40 °C. Die Mischung wird auf Eis gegeben,

mit Ammoniak alkalisch gemacht und mit Dichlormethan extrahiert. Die organischen Phasen werden getrocknet, eingedampft und der Rückstand an Kieselgel mit Dichlormethan/Methanol chromatographiert und kristallisiert, Ausbeute 217 mg (56 % der Theorie).

### Beispiel 4

### N-(2-Methyl-6-n-propyl-8α-ergolinyl)-formamid

849 mg 2-Methyl-6-n-propyl-8α-ergolinylamin (3 mmol) löst man in 40 ml Tetrahydrofuran und gibt 3,75 ml einer Mischung aus 6,15 ml 98%iger Ameisensäure, 12,5 ml Essigsäureanhydrid und 10 ml wasserfreiem Tetrahydrofuran zu. Nach 30 Minuten Rühren bei Raumtemperatur gibt man Eis zu, macht mit Ammoniak alkalisch und schüttelt mit Dichlormethan aus. Die organische Phase wird getrocknet, das Lösungsmittel abgedampft und der Rückstand an Kieselgel mit Essigester/Methanol chromatographiert und aus Diisopropylether kristallisiert, Ausbeute 647 mg (69 % der Theorie), [α]_{D} = + 30 °, (0,5 % in Chloroform).

Analog werden dargestellt:
aus 2,3β-Dihyro-2-methyl-6-n-propyl-8α-ergolinylamin das N-2,3β-Dihydro-2methyl6-n-propyl-8α-ergolinyl)-formamid
   Ausbeute 61 %
aus 2-Ethyl-6-n-propyl-8α-ergolinylamin das N-(2-Ethyl-6-n-propyl-8α-ergolinyl)-formamid
   Ausbeute 63 %
aus 9,10-Didehydro-2,3β-dihydro-2-methyl-6-n-propyl-8α-ergolinylamin das N-(9,10-Didehydro-2,3β-dihydro-2-methyl-6-n-propyl-8α-ergolinyl)-formamid
   Ausbeute 73 %
aus 6-Allyl-9,10-didehydro-2,3β-dihydro-2-methyl-8α-ergolinylamin das N-(6-Allyl-9,10-didehydro-2,3β-dihydro-2-methyl-8α-ergolinyl)-formamid
   Ausbeute 54 %
aus 9,10-Didehydro-2,3β-diyhdro-2-ethyl-6-n-propyl-8α-ergolinylamin das N-(9,10-Didehydro-2,3β-dihydro-2-ethyl-6-n-propyl-8α-ergolinyl)-formamid
   Ausbeute 71 %

### Beispiel 5

Analog Beispiel 3 werden erhalten:
aus N-(9,10-Didehydro-2,3-β-dihydro-2-methyl-6-n-propyl-8α-ergolinyl)formamid durch Oxidation mit tert.-Butylhypochlorit das N-(9,10-Didehydro-2-methyl-6-n-propyl-8α-ergolinyl)-formamid
   Ausbeute 66 %
aus N-(6-Allyl-9,10-didehydro-2,3β-dihydro-2-methyl-8α-ergolinyl)-formamid das N-(6-Allyl-9,10-didehydro-2-methyl-8α-ergolinyl)-formamid
   Ausbeute 48 %
aus N-(9,10-Didehydro-2,3β-diyhdro-2-ethyl-6-n-propyl-8α-ergolinyl)-formamid das
   N-(9,10-didehydro-2-ethyl-6-n-propyl-8α-ergolinyl)-formamid
   Ausbeute 63 %

### Beispiel 6

### N-(2-Methyl-6-n-propyl-8α-ergolinyl)-methoxyacetamid

849 mg 2-Methyl-6-n-propyl-8α-ergolinylamin (3 mmol) werden in 20 ml Pyridin gelöst und mit 2,28 ml Methoxyessigsäurechlorid (30 mmol) 30 Minuten bei Raumtemperatur gerührt. Dann wird auf Eis gegeben, 15 Minuten gerührt, mit Ammoniak alkalisch gemacht und mit Dichlormethan extrahiert. Die organische Phasen werden getrocknet und eingedampft, der Rückstand and Kieselgel mit Essigester/Methanol chromatographiert und aus Essigester kristallisiert, Ausbeute 429 mg (40 % der Theorie), [α]_{D} = + 24 ° (0,5 % in Chloroform).

### Beispiel 7

Durch Acylierung mit den entsprechenden Carbonsäurechloriden oder -anhydriden analog Beispiel 6 erhält man die folgenden Amide:
N-(2,3-Dihydro-2-methyl-6-n-propyl-8α-ergolinyl)-methoxyacetamid
   Ausbeute 63 %
N-(2-Methyl-6-n-propyl-8α-ergolinyl)-acetoxyacetamid
   Ausbeute 19 %, [α]_{D} = + 27 ° (0,5 % in Chloroform).
N-(9,10-Didehydro-2,3-dihydro-2-methyl-6-n-propyl-8α-ergolinyl)-acetoxyacetamid, Ausbeute 41 %.

### Beispiel 8

Analog Beispiel 3 erhält man aus N-(9,10-Didehydro-2,3-dihydro-2-methyl-6-n-propyl-8α-ergolinyl)-acetoxyacetamid durch Oxidation mit tert.-Butylhypochlorit N-(9,10-Didehydro-2-methyl-6-n-propyl-8α-ergolinyl)-acetoxyacetamid, Ausbeute 63 %.

### Beispiel 9

### N-(2-Methyl-6-n-propyl-8α-ergolinyl)-2-hydroxyacetamid

Man löst 530 mg N-(2-Methyl-6-n-propyl-8α-ergolinyl)-acetoxyacetamid (1,38 mmol) in 20 ml Methanol und 0,5 ml 7N KOH und rührt 15 Minuten bei Raumtemperatur. Dann wird mit Eis versetzt und mit Dichlormethan ausgeschüttelt. Die organischen Phasen werden getrocknet, eingedampft und aus Diisopropylether/Hexan kristallisiert, Ausbeute 321 mg (63 % der Theorie), [α]_{D} = + 20 ° (0,5 % in Chloroform).

Analog führt die Hydrolyse zu dem folgenden 2-Hydroxyacetamid:
N-(9,10-Didehydro-2-methyl-6-n-propyl-8α-ergolinyl)-2-hydroxyacetamid

### Beispiel 10

Analog Beispiel 6 werden durch Acylierung mit weiteren Carbonsäurechloriden die folgenden Amide erhalten:
N-(2-Methyl-6-n-propyl-8α-ergolinyl)-2-methyl-propionamid
   Ausbeute 21 %, [α]_{D} = + 24 ° (0,5 % in Chloroform)
N-(2-Ethyl-6-n-propyl-8α-ergolinyl)-2-methyl-propionamid
   Ausbeute 35 %
N-(9,10-Didehydro-2,3β-dihydro-2-methyl-6-n-propyl-8α-ergolinyl)-2-methylpropionamid, Ausbeute 42 %
N-(2-Methyl-6-n-propyl-8α-ergolinyl)-2-methyl-2-ethyl-butylamid
   Ausbeute 43 %, [α]_{D} = + 0,3 ° (0.5 % in Chloroform)
N-(2-Methyl-6-n-propyl-8α-ergolinyl)-trifluoracetamid
   Ausbeute 46 %. [α]_{D} = + 53 ° (0,5 % in Chloroform)
N-(2-Methyl-6-n-propyl-8α-ergolinyl)-methansulfonsäureamid
   Ausbeute 47 %, [α]_{D} = + 57 ° (0.5 % in Chloroform)
N-(2-Methyl-6-n-propyl-8α-ergolinyl)-3-(dimethylamino)-propionamid als weinsaures Salz, Ausbeute 60 %, [α]_{D} = + 1 ° (0,5 % in Methanol)

### Beispiel 11

Analog Beispiel 3 erhält man aus N-(9,10-Didehydro-2,3β-dihydro-2-methyl-6-npropyl-8α-ergolinyl)-2-methyl-propionamid durch Oxidation mit tert.-Butylhypochlorit N-(9,10-Didehydro-2-methyl-6-n-propyl-8α-ergolinyl)-2-methyl-propionamid, Ausbeute 72 %.

### Beispiel 12

### 1-Ethyl-3-(2-methyl-6-n-propyl-8α-ergolinyl)-harnstoff

Man löst 880 mg 2-Methyl-6-n-propyl-8α-ergolinylamin (3,1 mmol) in 100 ml Dichlormethan, gibt unter Eiskühlung 1,0 g 1,1-Carbonyldiimidazol (6,2 mmol) zu und rührt 2 Stunden bei Raumtemperatur. Dann gibt man 5 ml einer Lösung von Ethylamin in Dichlormethan /etwa 30 mmol) zu, rührt weitere 2 Stunden bei Raumtemperatur, versetzt mit Eis und Ammoniaklösung und schüttelt mit Dichlormethan aus. Die organischen Phasen werden getrocknet und eingedampft, der Rückstand an Kieselgel mit Dichlormethan/Methanol chromatographiert, Ausbeute 46 %, [α]_{D} = + 5 ° (0,5 % in Chloroform).

Analog werden mit 1,1'-Carbonyldiimidazol weitere Harnstoffe und Urethane und mit 1,1'Thiocarbonyldiimidazol Thioharnstoffe mit verschiedenen primären und sekundären Aminen oder Alkoholen dargestellt.
3-(9,10-Didehydro-2,3β-diyhdro-2-methyl-6-n-propyl-8α-ergolinyl)-1-ethyl-harnstoff, Ausbeute 81 %
1,1-Dimethyl-3-(2-methyl-6-n-propyl-8α-ergolinyl)-harnstoff, Ausbeute 65 %
1,1-Dimethyl-3-(2-ethyl-6-n-propyl-8α-ergolinyl)-harnstoff, Ausbeute 73 %
3-(2-Methyl-6-propyl-8α-ergolinyl)-1-morpholino-harnstoff, Ausbeute 67 %, [α]_{D} = + 15 ° (0,5 % in Chloroform)
1-Dimethylaminoethyl-1-ethyl-3-(2-methyl-6-n-propyl-8α-ergolinyl)-harnstoff, Ausbeute 23 % als weinsaures Salz, [α]_{D} = + 18° (0,5 % in Methanol)
3-(2-Methyl-6-n-propyl-8α-ergolinyl)-1,1-di-(2-trifluorethyl)-harnstoff Ausbeute 11 %
0-(3-Dimethylaminopropyl) -N-(2-methyl-6-n-propyl-8α-ergolinyl)-urethan Ausbeute 39 % als weinsaures Salz, [α]_{D} = + 9 ° (0,5 % in Methanol)
N-(2-Methyl-6-n-propyl-8α-ergolinyl)-carbamidsäuremethylester in 52 % Ausbeute, [α]_{D} = + 15 % (0,5 % in Chloroform)
3-(9,10-Didehydro-2,3β-dihydro-2-methyl-6-n-propyl-8α-ergolinyl)-1-dimethylaminoethyl-1-ethyl-harnstoff, Ausbeute 22 %
1,1-Dimethyl-3-(2-methyl-6-n-propyl-8α-ergolinyl)-thioharnstoff, Ausbeute 58 %
1-Ethyl-3-(2-methyl-6-n-propyl-8α-ergolinyl)-thioharnstoff, Ausbeute 73 %
1-Dimethylaminoethyl-1-ethyl-3-(2-methyl-6-n-propyl-8α-ergolinyl)-thioharnstoff Ausbeute 63 %

Ohne Zugabe eines Nucleophils erhält man mit 1,1'-Carbonyldiimidazol N-(2-Methyl-6-n-propyl-8α-ergolinyl)-1H-imidazol-1-carboxamid in 30 % Ausbeute, [α]_{D} = + 54 ° (0,5 % in Chloroform) und mit 1,1'-Thiocarbonyldiimidazol N-(2-Methyl-6-n-propyl-8α-ergolinyl)-isothiocyanat in 76 % Ausbeute, [α]_{D} = - 3 ° (0,5 % in Pyridin)

### Beispiel 13

Analog Beispiel 3 werden aus 3-(9,10-Didehydro-2,3β-dihydro-2-methyl-6-n-propyl-8α-ergolinyl)-1-ethyl-harnstoff durch Oxidation mit tert.-Butylhypochlorit 3-(9,10-Didehydro-2-methyl-6-n-propyl-8α-ergolinyl)-1-ethylharnstoff, Ausbeute 43 %

und aus 3-(9,10-Didehydro-2,3β-dihydro-2-methyl-6-n-propyl-8α-ergoliny)-1-dimethylaminomethyl-1-ethyl-harnstoff 3-(9,10-Didehydro-2-methyl-6-n-propyl-8αergolinyl)-1-dimethylaminoethyl-1-ethyl-harnstoff, Ausbeute 66 % erhalten.

### Beispiel 14

### N-(2-Methyl-6-n-propyl-8α-ergolinyl)-2-methyl-thiopropionsäureamid

Man suspendiert 353 mg N-(Methyl-6-n-propyl-8α-ergolinyl)-2-methylpropionamid (1 mmol) in 20 mnl Toluol, gibt 404 mg 2,4-Bis-(4-dimethoxyphenyl-1,2,3,4-dithiadiphosphetan-2,4-disulfid (2 mmol) (Lawessons Reagenz) zu und erhitzt 3 Stunden auf 100 °C. Nach dem Abkühlen fügt man Wasser zu, macht mit Ammoniak alkalisch und schüttelt mit Dichlormethan aus. Die organischen Phasen werden getrocknet und eingedampft, der Rückstand an Kieselgel mit Dichlormethan/ Methanol chromatographiert, und aus Essigester/Diisopropylether/Hexan kristallisiert, Ausbeute 55 %, [α]_{D} = + 116 ° (0,5 % in Chloroform).

Analog werden die folgenden Thioamide aus den Amiden dargestellt:
N-(2-Methyl-6-n-propyl-8α-ergolinyl)-thioformamid
   Ausbeute 55%, [α]_{D} = + 123° (0,5 % in Chloroform)
N-(2-Methyl-6-n-propyl-8α-ergolinyl)-methoxy-thioacetamid,
   Ausbeute 68 %, [α]_{D} = + 101° (0.5 % in Chloroform)
N-(2-Methyl-6-n-propyl-8α-ergolinyl)-2-mercapto-thioacetamid,
   Ausbeute 46 %, [α]_{D} = - 70° (0,2 % in Chloroform)

N-(2-Hethyl-6-n-propyl-8α-ergolinyl)-2-acetoxy-thioacetamid in 15 % Ausbeute, [α]_{D} = + 121 = (0.5 % in Chloroform). Daraus erhalt man durch Verseifung analog Beispiel 9 N-(2-Methyl-6-n-propyl-8α-ergolinyl)-2-hydroxythioacetamid in 58 % Ausbeute, [α]_{D} = + 68 ° (0,5 % in Chloroform).
N-(2-Methyl-6-n-propyl-8α-ergolinyl)-3-(dimethylamino)-thiopropionamid, Ausbeute 18 %, [α]_{D} = + 83 ° (0,25 % in Chloroform)
N-(9,10-Didehydro-2-methyl-6-n-propyl-8α-ergolinyl)-2-methyl-thiopropionsaureamid, Ausbeute 34 %
N-(2-Methyl-6-n-propyl-8α-ergolinyl)-2-ethyl-2-methyl-thiobuttersäureamid Ausbeute 54 %

### Beispiel 15

### 1-Ethyl-3-(2-methyl-6-n-propyl-8α-ergolinyl)-thioharnstoff

283 mg 2-Methyl-6-n-propyl-8α-ergolinylamin (1 mmol) werden in 5 ml Dichlormethan gelöst und mit 150 mg Ethylisothiocyanat (2 mmol) in 5 ml Dichlormethan versetzt. Man rührt 2 Stunden bei Raumtemperatur, versetzt dann mit Wasser und Ammoniak und schuttelt mit Dichlormethan aus. Die organischen Phasen werden getrocknet und eingedampft, der Rückstand an Kieselgel mit Dichlormethan/Methanol chromatographiert und kristallisiert. Ausbeute 34 %, [α]_{D} = + 34 ° (0,5 % in Chloroform).

Analog wird mit Methylisocyanat der 1-Methyl-3-(2-methyl-6-n-propyl-8α-ergolinyl)-harnstoff dargestellt, Ausbeute 53 %.

### Beispiel 16

### (2-Methoxymethyl-6-n-propyl-8α-ergolinyl)-formamid

1,1 g 2-Morpholinomethyl-6-n-propyl-8α-ergolinylamin (3 mmol) werden wie in Beispiel 4 beschrieben formyliert, dann in 50 ml Tetrahydrofuran, mit 2 ml Methyliodid versetzt und 20 Stunden bei Raumtemperatur geruhrt. Anschließend wird im Eisbad gekühlt und zur Vervollständigung der Kristallisation mit Diisopropylether versetzt. Die Kristalle werden isoliert und in 30 ml Methanol gelöst. Gleichzeitig stellt man sich aus 230 mg Natrium (10 mmol) in 20 ml Methanol eine Methylatlösung dar, in die man die Lösung des Quartärsalzes bei 0 °C eintropft. Nach 15 Minuten Rühren bei 0 °C und 30 Minuten bei Raumtemperatur gibt man Eis zu und extrahiert mit Dichlormethan. Die organischen Phasen werden getrocknet und eingedampft, der Rückstand an Kieselgel mit Dichlormethan und Methanol chromatographiert, Ausbeute 470 mg (48 % der Theorie).

### Beispiel 17

### N-(2-Methylthiomethyl-6-n-propyl-8α-ergolinyl)-formamid

Verwendet man Natriummethanthiolat als Nukleophil analog Beispiel 16, so erhält man in Dichlormethan die Titelverbindung in 37 % Ausbeute.

### Beispiel 18

### N-(6-n-Propyl-2-vinyl-8α-ergolinyl)-formamid

1,1 g 2-Morpholinomethyl-6-n-propyl-8α-ergolinylamin (3 mmol) werden wie in Beispiel beschrieben formyliert, dann in 50 ml Tetrahydrofuran und 1 ml Triethylamin gelöst, auf - 40 °C gekühlt und mit einer Lösung von 0,5 ml tert.-Butylhypochlorit in 10 ml Tetrahydrofuran versetzt. Nach 30 Minuten Rühren gießt man den Ansatz auf Eis, macht mit Ammoniak alkalisch und extrahiert mit Dichlormethan. Die organischen Phasen werden getrocknet und eingedampft. Der Rückstand wird in 40 ml Tetrahydrofuran gelöst und zu einem Wittigreagenz gegeben, das aus 6 g Methyl-triphenylphosphoniumbromid und 2 g Kalium-tert.-butylat in 70 ml Tetrahydrofuran durch 15 Minuten Rühren bei - 78 °C bereitet wurde. Man läßt 1 Stunde bei - 78 °C reagieren, erwärmt dann langsam auf - 40 °C und rührt 2 Stunden bei - 40 °C. Anschließend wird mit Kochsalzlösung versetzt, mit Essigester extrahiert und die organischen Phasen getrocknet und eingedampft. Der Rückstand wird an Kieselgel mit Dichlormethan/Methanol chromatographiert und kristallisiert, Ausbeute 35 %.

### Beispiel 19

Man löst 470 mg N-(2-Methyl-6-n-propyl-8α-ergolinyl)-isothiocyanat (1.4 mmol) in 10 ml Dichlormethan, gibt 2,31 ml N'-Ethyl-N,N'-dimethylethylendiamin (14 mmol) zu und rührt bei Raumtemperatur über Nacht. Dann wird mit Eis verdünnt, mit Ammoniak versetzt und mit Dichlormethan extrahiert. Der Rückstand wird an Aluminiumoxid mit Dichlormethan chromatographiert und aus Essigester/Diisopropylether kristallisiert. Man erhält 415 mg 1-Ethyl-1-(2-dimethylaminoethyl)-3-(2-methyl-6-n-propyl-8α-ergolinyl)-thioharnstoff (65 % der Theorie), [α]_{D} = + 50 ° (0,5 % in Chloroform).

Mit anderen Nukleophilen werden die folgenden Derivate dargestellt:
N-(2-Methyl-6-n-propyl-8α-ergolinyl)-thiocarbamidsäure-O-(3-dimethylaminopropyl)-ester in 14 % Ausbeute, [α]_{D} = + 40 ° (0,5 % in Chloroform)
N-(2-Methyl-6-n-propyl-8α-ergolinyl)-dithiocarbamidsäuremethylester in 36 % Ausbeute, [α]_{D} = + 86 ° (0,5 % in Chloroform)

## Patentansprüche

1. Verbindungen der Formel I 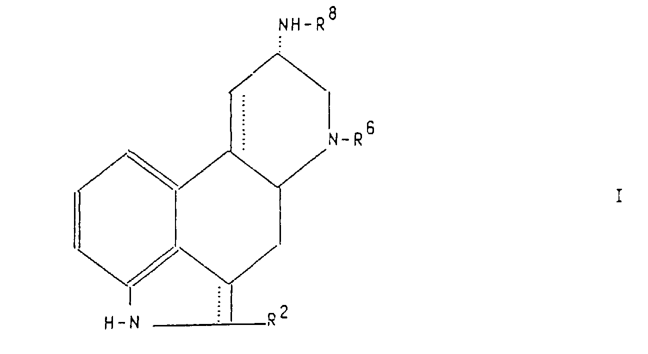 worin
C 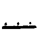 C jeweils eine Einfach- oder Doppelbindung,
R² gegebenenfalls substituiertes C₁₋₇-Alkyl, C₂₋₇-Alkenyl, CH₂-O- C₁₋₄-Alkyl oder CH₂-S-C₁₋₄-Alkyl.
R⁶ C₂₋₆-Alkyl, C₃₋₆-Alkenyl oder C₃₋₅-Cycloalkyl-C₁₋₂-alkyl und
R⁸ -CXR³, oder -SO₂R⁵ ist,
worin R³ Wasserstoff, gegebenenfalls substituiertes C₁₋₆-Alkyl, S-C₁₋₆-Alkyl, -0-(CH₂)ₙ-N(CH₃)₂ oder NR⁹R¹⁰ ist, X Sauerstoff oder Schwefel ist und R⁵ CH₃ oder eine gegebenenfalls mit C₁₋₄-Alkyl mono- oder disubstituierte Aminogruppe bedeutet, worin R⁹ und R¹⁰ jeweils Wasserstoff, C₁₋₄-Alkyl, C₃₋₄-Alkenyl, -(CH₂)ₙN(CH₃)₂, CH₂CF₃ oder gemeinsam mit dem Stickstoffatom einen 5-6 gliedrigen gesättigten oder ungesättigten Heterocyclus bilden, der durch ein bis zwei weitere Heteroatome unterbrochen sein kann und n = 1,2,3 oder 4 ist sowie deren Säureadditionssalze, wobei R⁸ nicht CXN (C₂H₅)₂ bedeutet und falls R⁸ -CO-C(CH₃)₃ und R⁶ n-Propyl und R² Methyl ist, C₂ 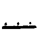 C₃ eine Einfachbindung bedeutet.

2. (Dimethylamino)-(2-methyl-6-n-propyl-8α-ergolinylamino)-sulfon
(9,10-Didehydro-2-methyl-6-n-propyl-8α-ergolinyl)-dimethylaminosulfon
N-(2-Methyl-6-n-propyl-8α-ergolinyl)-formamid
N-(9,10-Didehydro-2-methyl-6-n-propyl-8α-ergolinyl)-formamid
N-(2-Methyl-6-n-propyl-8α-ergolinyl)-methoxyacetamid
N-(2-Methyl-6-n-propyl-8α-ergolinyl)-acetoxyacetamid
N-(2-Methyl-6-n-propyl-8α-ergolinyl)-2-hydroxyacetamid
N-(2-Methyl-6-n-propyl-8α-ergolinyl)-2-methyl-propionamid
1-Ethyl-3-(2-methyl-6-n-propyl-8α-ergolinyl)-harnstoff
3-(2-Methyl-6-n-propyl-8α-ergolinyl)-1-morpholino-harnstoff
3-(9,10-Didehydro-2-methyl-6-n-propyl-8α-ergolinyl)-1-dimethylaminoethyl1-ethyl-harnstoff
N-(2-Methyl-6-n-propyl-8α-ergolinyl)-2-methyl-thiopropionsäureamid
1-Ethyl-3-(2-methyl-6-n-propyl-8α-ergolinyl)-thioharnstoff
N-(2-Methoxymethyl-6-n-propyl-8α-ergolinyl)-formamid
N-(6-n-Propyl-2-vinyl-8α-ergolinyl)-formamid
N-(2-Methyl-6-n-propyl-8α-ergolinyl)-dithiocarbamidsäuremethylester nach Anspruch 1.

3. N-(2-Methyl-6-n-propyl-8α-ergolinyl)-carbamidsäuremethylester

4. N-(2-Methyl-6-n-propyl-8α-ergolinyl)-trifluoracetamid

5. N-(2-Methyl-6-n-propyl-8α-ergolinyl)-2-mercapto-thioacetamid

6. Arzneimittel enthaltend mindestens eine Verbindung gemäß den Ansprüchen 1 und 2.

7. Verfahren zur Herstellung der Verbindungen der Formel I gemäß Anspruch 1, indem man
a) eine Verbindung der Formel II 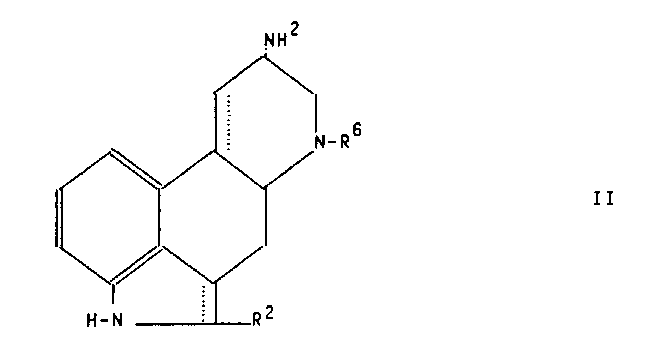 worin R² und R⁶ die obige Bedeutung haben, mit einer Säure oder deren funktionellem Derivat acyliert,
b) eine Verbindung der Formel II und eine Verbindung der Formel III
R⁹-N=C=X III
worin R⁹ und X die obige Bedeutung haben, R⁹ jedoch nicht Wasserstoff sein kann, addiert zu einer Verbindung mit R⁸ in der Bedeutung von -CX-NHR⁹,
c) eine Verbindung der Formel IV 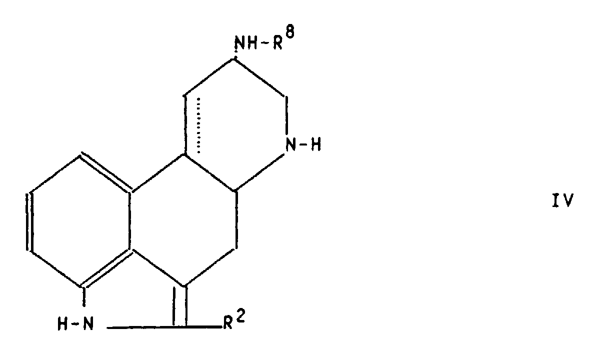 worin R² und R⁸ die obige Bedeutung haben, alkyliert oder alkenyliert
d) eine Verbindung der Formel V 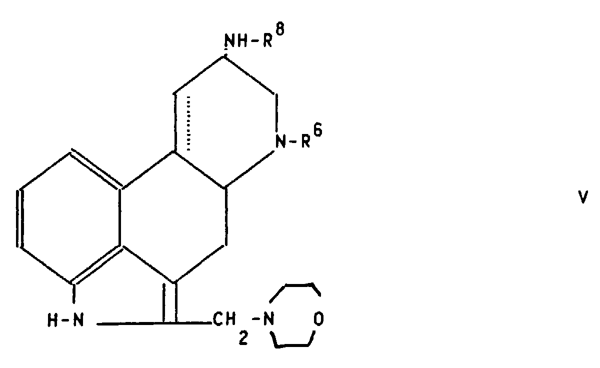 worin R⁶ und R⁸ die obige Bedeutung haben in 2-Stellung substituiert
e) eine Verbindung der Formel VI 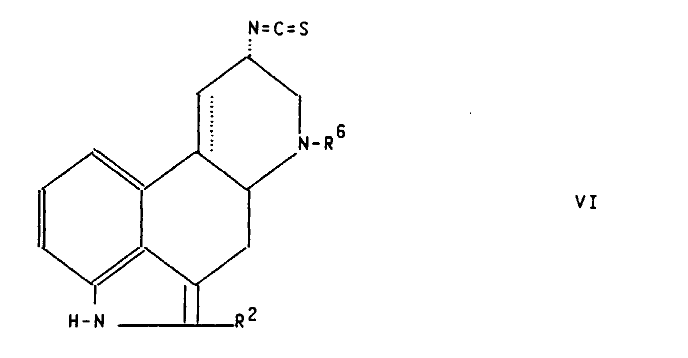 worin R² und R⁶ die oben genannte Bedeutung haben mit einem Nukleophil umsetzt zu Verbindungen der Formel I mit R³ = -S-C₁₋₆-Alkyl, -O-(CH₂)ₙ-N(CH₃)₂ oder NR⁹R¹⁰, worin R⁹, R¹⁰ und n die oben genannte Bedeutung haben und gewünschtenfalls anschließend eine C 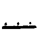 C-Einfachbindung oxidiert oder eine C 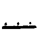 C-Doppelbindung reduziert oder eine Carbonylgruppe thioliert oder die Säureadditionssalze bildet.

8. Verbindungen der Formel II 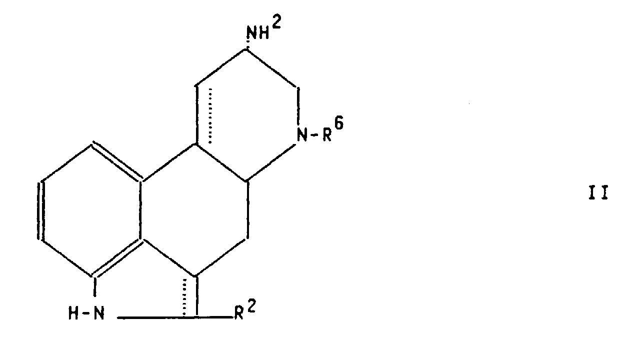 worin R² und R⁶ die obige Bedeutung haben.

9. Verbindungen der Formel VI 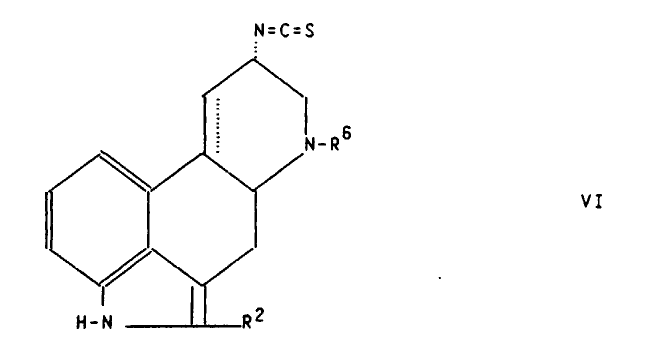 worin R² und R⁶ die obige Bedeutung haben.

## Claims

1. Compounds of formula I 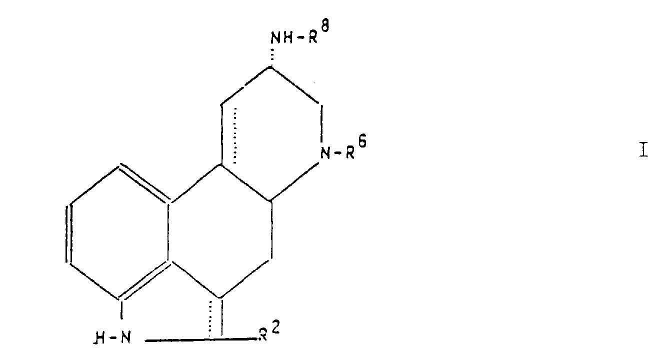 in which
C 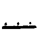 C is in each case a single bond or a double bond,
R² is optionally substituted C₁₋₇alkyl, C₂₋₇alkenyl, CH₂-O-C₁₋₄alkyl or CH₂-S-C₁₋₄alkyl,
R⁶ is C₂₋₆alkyl, C₃₋₆alkenyl or C₃₋₅cycloalkyl-C₁₋₂alkyl, and
R⁸ is -CXR³ or -SO₂R⁵,
in which R³ is hydrogen, optionally substituted C₁₋₆alkyl, S-C₁₋₆alkyl, -O-(CH₂)ₙ-N(CH₃)₂ or NR⁹R¹⁰, X is oxygen or sulphur and R⁵ represents CH₃ or an amino group that is optionally mono- or di-substituted by C₁₋₄alkyl, in which R⁹ and R¹⁰ each represent hydrogen, C₁₋₄alkyl, C₃₋₄alkenyl, -(CH₂)ₙ-N(CH₃)₂ or CH₂CF₃ or together with the nitrogen, atom form a 5- or 6-membered saturated or unsaturated heterocycle that may be interrupted by one or two further hetero atoms, and n is 1, 2, 3 or 4,
and their acid addition salts,
wherein R⁸ does not represent CXN(C₂H₅)₂, and when R⁸ is -CO-C(CH₃)₃ and R⁶ is n-propyl and R² is methyl, C₂ 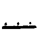 C₃ represents a single bond.

2. Dimethylamino 2-methyl-6-n-propyl-8α-ergolinylamino sulphone
9,10-didehydro-2-methyl-6-n-propyl-8α-ergolinyldimethylamino sulphone
N-(2-methyl-6-n-propyl-8α-ergolinyl)-formamide
N-(9,10-didehydro-2-methyl-6-n-propyl-8α-ergolinyl)formamide
N-(2-methyl-6-n-propyl-8α-ergolinyl)-methoxyacetamide
N-(2-methyl-6-n-propyl-8α-ergolinyl)-acetoxyacetamide
N-(2-methyl-6-n-propyl-8α-ergolinyl)-2-hydroxyacetamide
N-(2-methyl-6-n-propyl-8α-ergolinyl)-2-methyl-propionamide
1-ethyl-3-(2-methyl-6-n-propyl-8α-ergolinyl)-urea
3-(2-methyl-6-n-propyl-8α-ergolinyl)-1-morpholino-urea
3-(9,10-didehydro-2-methyl-6-n-propyl-8α-ergolinyl)-1-dimethylaminoethyl-1-ethyl-urea
N-(2-methyl-6-n-propyl-8α-ergolinyl)-2-methyl-thiopropionic acid amide
1-ethyl-3-(2-methyl-6-n-propyl-8α-ergolinyl)-thiourea N-(2-methoxymethyl-6-n-propyl-8α-ergolinyl)-formamide
N-(6-n-propyl-2-vinyl-8α-ergolinyl)-formamide
N-(2-methyl-6-n-propyl-8α-ergolinyl)-dithiocarbamic acid methyl ester
according to claim 1.

3. N-(2-methyl-6-n-propyl-8α-ergolinyl)-carbamic acid methyl ester.

4. N-(2-methyl-6-n-propyl-8α-ergolinyl)-trifluoroacetamide.

5. N-(2-methyl-6-n-propyl-8α-ergolinyl)-2-mercaptothioacetamide.

6. Medicament comprising at least one compound according to claims 1 and 2.

7. Process for the preparation of compounds of formula I according to claim 1, by
a) acylating a compound of formula II 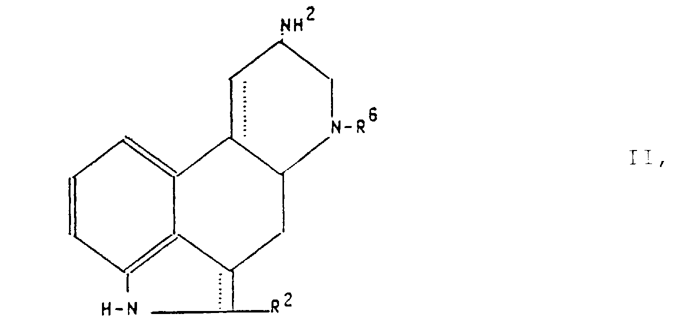 in which R² and R⁶ are as defined above, with an acid or a functional derivative thereof,
b) adding a compound of formula II and a compound of formula III
R⁹-N=C=X III,
in which R⁹ and X are as defined above but R⁹ may not be hydrogen, to form a compound in which R⁸ represents -CX-NHR⁹,
c) alkylating or alkenylating a compound of formula IV 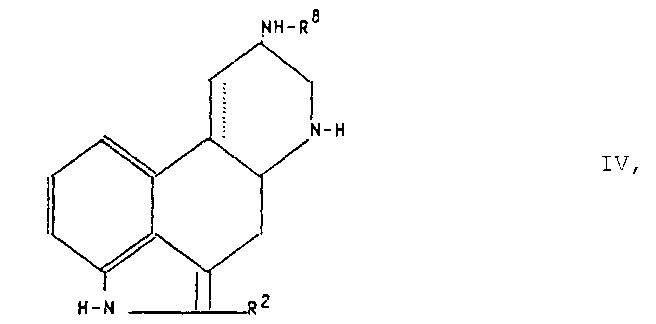 in which R² and R⁸ are as defined above,
d) substituting in the 2-position a compound of formula V 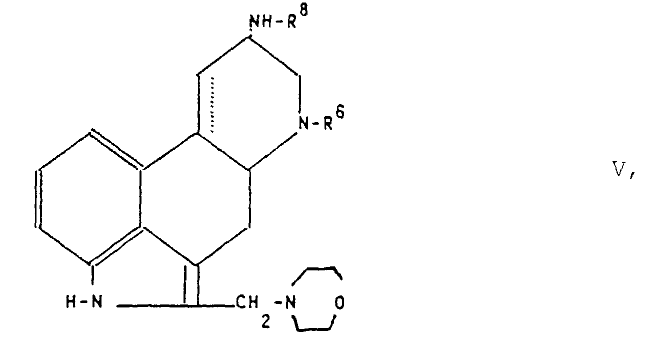 in which R⁶ and R⁸ are as defined above,
e) reacting a compound of formula VI 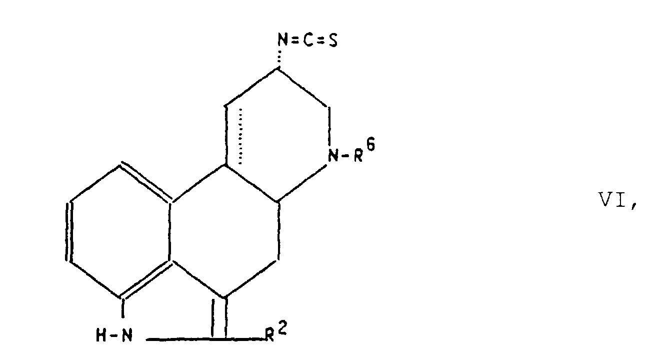 in which R² and R⁶ are as defined above, with a nucleophilic reagent to form compounds of formula I in which R³ represents -S-C₁₋₆alkyl, -O-(CH₂)ₙ-N(CH₃)₂ or NR⁹R¹⁰, in which R⁹, R¹⁰ and n are as defined above, and then, if desired, oxidising a C 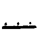 C single bond or reducing a C 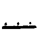 C double bond or thiolating a carbonyl group or forming the acid addition salts.

8. Compounds of formula II 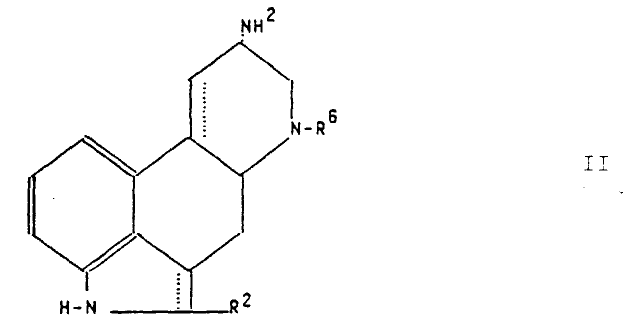 in which R² and R⁶ are as defined above.

9. Compounds of formula VI 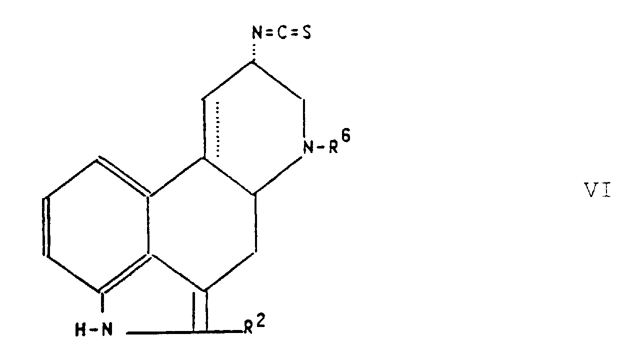 in which R² and R⁶ are as defined above.

## Revendications

1. Composés de formule I 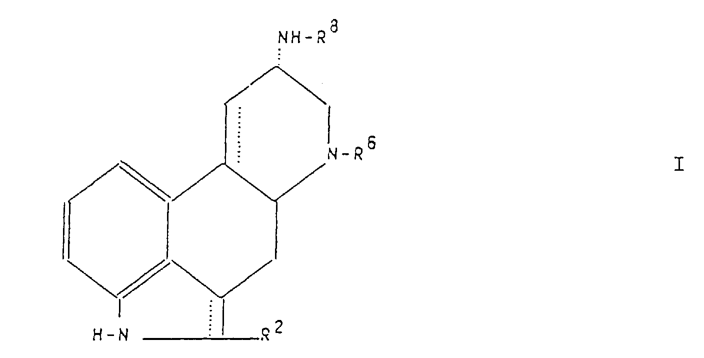 dans laquelle
C 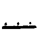 C représente chaque fois une simple ou une double liaison,
R² représente alkyle en C₁-C₇, alcényle en C₂-C₇, CH₂-O-(alkyle en C₁-C₄) ou CH₂-S-(alkyle en C₁-C₄) éventuellement substitué,
R⁶ représente alkyle en C₂-C₆, alcényle en C₃-C₆ ou (cycloalkyle en C₃-C₅)-alkyle en C₁-C₂ et
R⁸ représente -CXR³ ou -SO₂R⁵,
où R³ représente l'hydrogène, alkyle en C₁-C₆ éventuellement substitué, S-alkyle en C₁-C₆, -O-(CH₂)ₙ-N(CH₃)₂ ou NR⁹R¹⁰, X représente l'oxygène ou le soufre et R⁵ représente CH₃ ou un groupe amino éventuellement mono- ou disubstitué par alkyle en C₁-C₄, où R⁹ et R¹⁰ chacun représentent l'hydrogène, alkyle en C₁-C₄, alcényle en C₃-C₄, -(CH₂)ₙN(CH₃)₂, CH₂CF₃ ou ensemble avec l'atome d'azote forment un hétérocycle à 5 ou 6 chaînons saturé ou insaturé, qui peut être interrompu par un à deux autres hétéroatomes et n = 1, 2, 3 ou 4 ainsi que des sels d'addition d'acide où R⁸ n'étant pas CXN(C₂H₅)₂ et si R⁸ représente CO-C(CH₃)₃ et R⁶ représente n-propyle et R² représente méthyle, C₂ 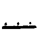 C₃ représente une simple liaison.

2. (Diméthylamino)-(2-méthyl-6-n-propyl-8α-ergolinylamino)-sulfone
(9,10-didéshydro-2-méthyl-6-n-propyl-8α-ergolinyl)diméthylamino-sulfone
N-(2-méthyl-6-n-propyl-8α-ergolinyl)-formamide
N-(9,10-didéshydro-2-méthyl-6-n-propyl-8α-ergolinyl)formamide
N-(2-méthyl-6-n-propyl-8α-ergolinyl)-méthoxyacétamide
N-(2-méthyl-6-n-propyl-8α-ergolinyl)-acétoxyacétamide
N-(2-méthyl-6-n-propyl-8α-ergolinyl)-2-hydroxyacétamide
N-(2-méthyl-6-n-propyl-8α-ergolinyl)-2-méthylpropionamide
1-éthyl-3-(2-méthyl-6-n-propyl-8α-ergolinyl)-urée
3-(2-méthyl-6-n-propyl-8α-ergolinyl)-1-morpholino-urée
3-(9,10-didéshydro-2-méthyl-6-n-propyl-8α-ergolinyl)-1-diméthylaminoéthyl-1-éthyl-urée
N-(2-méthyl-6-n-propyl-8α-ergolinyl)-2-méthylthiopropionamide
1-éthyl-3-(2-méthyl-6-n-propyl-8α-ergolinyl)-thiourée
N-(2-méthoxyméthyl-6-n-propyl-8α-ergolinyl)-formamide
N-(6-n-propyl-2-vinyl-8α-ergolinyl)-formamide
N-(2-méthyl-6-n-propyl-8α-ergolinyl)-dithiocarbamidate de méthyle selon la revendication 1.

3. N-(2-Méthyl-6-n-propyl-8α-ergolinyl)-carbamidate de méthyle.

4. N-(2-Méthyl-6-n-propyl-8α-ergolinyl)trifluoracétamide.

5. N-(2-Méthyl-6-n-propyl-8α-ergolinyl)-2-mercaptothioacétamide.

6. Médicament contenant au moins un composé selon les revendications 1 et 2.

7. Procédé pour la préparation de composés de formule I selon la revendication 1
a) par acylation d'un composé de formule II 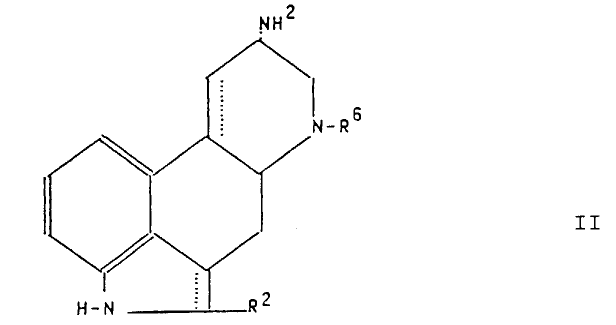 dans laquelle R² et R⁶ ont la signification donnée ci-dessus, avec un acide ou son dérivé fonctionnel,
b) par addition d'un composé de formule II et d'un composé de formule III
R⁹-N=C=X III
dans laquelle R⁹ et X ont la signification donnée ci-dessus, cependant R⁹ ne peut pas être l'hydrogène, a un composé avec R⁸ ayant la signification de -CX-NHR⁹,
c) par alkylation ou alcénylation d'un composé de formule IV 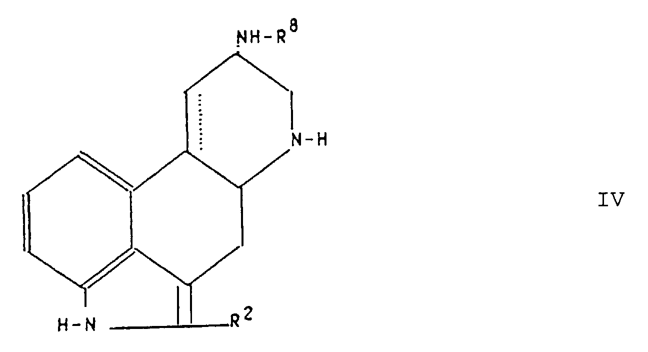 dans laquelle R² et R⁸ ont la signification donnée ci-dessus,
d) par substitution en position 2 d'un composé de formule V 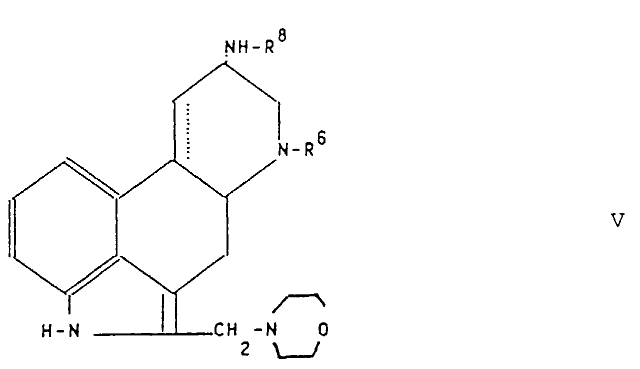 dans laquelle R⁶ et R⁸ ont la signification donnée ci-dessus,
e) par réaction d'un composé de formule VI 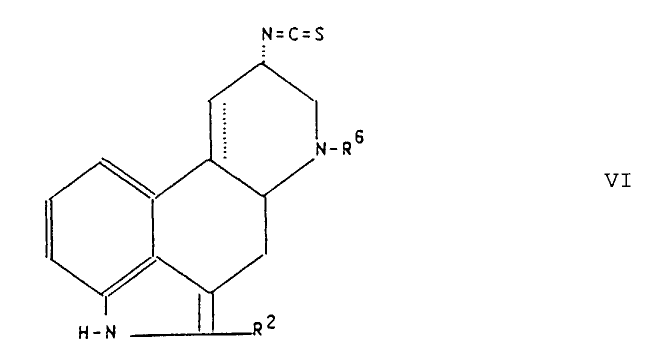 dans laquelle R² et R⁶ ont la signification donnée ci-dessus, avec un nucléophile pour obtenir des composés de formule I avec R³ = -S-(alkyle en C₁-C₆), -O-(CH₂)ₙ-N(CH₃)₂ ou NR⁹R¹⁰, dans laquelle R⁹, R¹⁰ et n ont la signification donnée ci-dessus et si on le souhaite on oxyde ensuite une simple liaison C 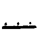 C ou on réduit une double liaison C 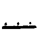 C ou on effectue une thiolation d'un groupe carbonyle ou on forme des sels d'addition d'acides.

8. Composés de formule II 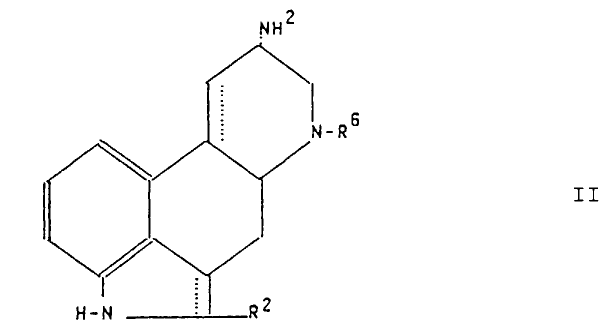 dans laquelle R² et R⁶ ont la signification donnée ci-dessus.

9. Composés de formule VI 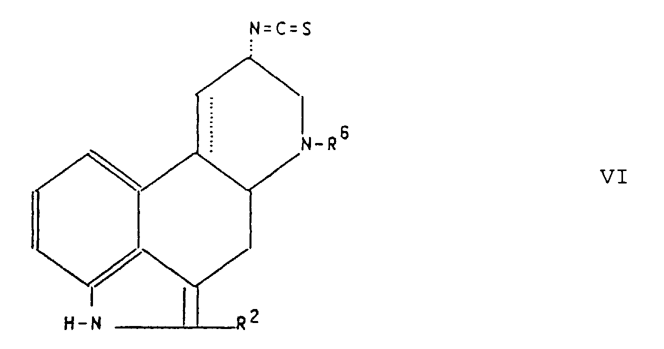 dans laquelle R² et R⁶ ont la signification donnée ci-dessus.
